## Europäisches Patentamt
## European Patent Office
## Office européen des brevets

(11) Veröffentlichungsnummer: **0 157 308**
**A2**

(12) ## EUROPÄISCHE PATENTANMELDUNG

(21) Anmeldenummer: 85103379.5

(22) Anmeldetag: **22.03.85**

(51) Int. Cl.⁴: **G 01 N 25/04**

(30) Priorität: **31.03.84  DE 3412024**

(43) Veröffentlichungstag der Anmeldung: **09.10.85**
Patentblatt 85/41

(84) Benannte Vertragsstaaten: **AT BE CH FR GB IT LI LU NL SE**

(71) Anmelder: **Fritz Winter, Eisengiesserei O.H.G., Albert-Schweitzer-Strasse, D-3570 Stadtallendorf 1 (DE)**

(72) Erfinder: **Lampic-Opländer, Milan, Dr., Schwanallee 21, D-3550 Marburg/Lahn (DE)**

(74) Vertreter: **Gudel, Diether, Dr. et al, Patentanwälte Dr. V. Schmied-Kowarzik Dipl.-Ing. G. Dannenberg Dr. P. Weinhold Dr. D. Gudel Dipl.-Ing. S. Schubert Dr. P. Barz Grosse Eschenheimer Strasse 39, D-6000 Frankfurt am Main 1 (DE)**

(54) Verfahren und Vorrichtung zur thermischen Analyse von Gusseisen.

(57) Beschrieben wird ein Verfahren und eine Vorrichtung zur thermischen Analyse von Gusseisen, wobei man eine flüssige Probe des zu untersuchenden Gusseisens in einen ersten Probentiegel (1) mit einem Temperatur-Messfühler (6) gibt und gleichzeitig in einen zweiten Probentiegel (3), ebenfalls mit einem Temperatur-Messfühler (7). Die Wand des zweiten Probentiegels (3) ist mit einem Sauerstoff abgebenden Mittel beschichtet. Man misst die Abkühlungskurven in beiden Probentiegeln (1, 3) und simuliert dadurch die Erstarrung des Gusseisens in einer realen Form.

EP 0 157 308 A2

## Beschreibung

Die Erfindung betrifft ein Verfahren zur thermischen Analyse von Gußeisen, wobei man eine Probe des zu untersuchenden Gußeisens in einen Probentiegel mit einem Temperatur-Meßfühler gibt und die Abkühlung der Probe über der Zeit mißt. Außerdem bezieht sich die Erfindung auf eine Vorrichtung zur Durchführung dieses Verfahrens mit einem Probentiegel mit einem Temperatur-Meßfühler.

Bei einem bekannten Verfahren zur thermischen Analyse von Gußeisen wird die Schmelze in einen Einwegtiegel aus kunstharzgebundenem Sand mit eingebauten Meßfühler (Thermoelement) gegossen. Die im Tiegel erkaltende Schmelze liefert eine Abkühlungskurve, die entweder mit einem Analogschreiber aufgezeichnet oder in einem Rechner digital umgesetzt und anschließend als Rechner-Plott ausgegeben wird.

Bestimmte, bei der Abgabe der Kristallisationswärme auftretende Effekte auf der Abkühlungskurve werden hierbei entweder zur Bestimmung der Gehalte an Kohlenstoff und Silizium des Eisens herangezogen (in diesem Fall enthält der Tiegel eine bestimmte Menge von metallischem Tellur) oder zur Ermittlung von Art und Mengenanteilen der kristallisierten Phasen, bei gleichzeitiger Bestimmung einer summarischen Größe zur Bewertung der chemischen Zusammensetzung (Sättigungsgrad $S_c$ oder Kohlenstoff-Liquidus-Äquivalent CEL ). Der Tiegel enthält keinen Zusatzstoff und das Verfahren wird vorzugsweise in Form einer "derivativen Differential-Thermoanlayse" (DDTA) durchgeführt.

0157308

Diese Analyse ist in ihren Grundzügen erläutert in der Dissertation von Dipl.-Ing. Milan Lampic, T.U. Berlin, 1978 mit dem Titel "Entwicklung und Erprobung eines Meßverfahrens zur Erfassung der Kristallisation der Randschicht von Gußstücken". Bei der DDTA bildet man die Differenz zwischen den Abkühlungskurven von zwei Stoffen, von denen nur einer in einem gegebenen Temperaturbereich Phasenumwandlungen erleidet. Die somit erhaltene Differenzkurve (DTA) wird differenziert. Die damit erhaltene DDTA-Kurve ergibt insbesondere in ihren Spitzen eine gute Anzeige für stattgefundene Phasenumwaldlungen und andere interessierende Vorgänge. Dies wird weiter unten näher erläutert.

Sowohl die gewöhnliche Zeit-Temperatur-Abkühlungskurve als auch die DDTA-Kurve kennzeichnen nur den Zustand des Eisens im beispielsweise 300 g fassenden Tiegel und sagen so gut wie nichts aus über das Verhalten des Eisens beim Gießen in häufig sehr kompliziert gebaute, ein Vielfaches davon fassende, in der Regel feuchte, tongebundene Sandformen zur Herstellung realer Gußstücke.

Zur Steuerung des Erstarrungsablaufes solcher Gußstücke (z.B. Zylinderblöcke) mit dem Ziel des Erreichens bestimmter Stückeigenschaften, insbesondere aber der Vermeidung von verdeckten Fehlern, ist jedoch eine Vorprobe erforderlich, die das Verhalten des Eisens beim Gießen mit einer möglichst hohen Wahrscheinlichkeit vorherzusagen erlaubt.

Der Ausgangszustand des Gußeisens unmittelbar vor dem Vergießen wird im wesentlichen bestimmt durch:

- 3 -

0157308

- chemische Zusammensetzung, unter besonderer Berücksichtigung der Gehalte an höher sauerstoffaffinen Elementen, wie Aluminium und Titan, sowie an Sauerstoff und Stickstoff;

- Schmelzbedingungen z.B. gegeben durch Einsatzmaterial, Art des Ofens, Zeit- und Temperaturprogramm des Schmelzens, Schlackenführung usw.;

- Bedingungen beim Transport und Speichern.

Die sich daraus ergebenden Parameter für den Kristallisationsablauf sind:

- Struktur des flüssigen Zustandes, gegeben durch Atomnah- und fernordnung;

- Art, Mengen und Größe der als Kristallisationskeime in Frage kommenden nichtmetallischen Einschlüsse unter besonderer Berücksichtigung der Bindungsart des Sauerstoffs sowie die sogenannte Desoxidationsreserve.

Der Erfindung liegt die Aufgabe zugrunde, ein Verfahren und eine Vorrichtung der eingangs genannten Art vorzuschlagen, mit dem auf einfache Weise die Erstarrung des Gußeisens in einer realen Form mit ausreichender Genauigkeit simuliert werden kann.

Zur Lösung dieser Aufgabe ist das erfindungsgemäße Verfahren dadurch gekennzeichnet, daß man dieselbe Probe gleichzeitig in einen zweiten Probentiegel mit einem zweiten Temperatur-Meßfühler gibt, dessen Innenwand zumindest teilweise mit einem Sauerstoff abgebenden Mittel beschichtet ist.

Die Art dieses Sauerstoff abgebenden Mittels und seine dem flüssigen Gußeisen zugewandte Fläche dimensioniert man so, daß dort in etwa soviel Sauerstoff an die Probe abgegeben wird, wie dies beim Gießen in der realen Form der Fall wäre. Erfahrungsgemäß handelt es sich dabei um 20 bis 40 ppm $O_2$. Durch das erfindungsgemäße Verfahren kann man daher die Erstarrung des Gußeisens in der bekannten, realen Form mit hoher Genauigkeit simulieren. In beiden Probentiegeln mißt man Abkühlungskurven (DTA-Kurven und/oder DDTA-Kurven). Daraus lassen sich die Regeln für metallurgisches Handeln (Schmelzbehandlung, Desoxidation, Impfen etc.) ableiten.

Hierbei berücksichtigt man somit, daß je nach Größe der Sauerstoffaufnahme beim Gießen und Art der Bindung dieses Sauerstoffs sich für den Erstarrungsablauf der Schmelze unterschiedliche Bedingungen ergeben, die das Ergebnis beim realen Gießen innerhalb weiter Grenzen beeinflussen können. Diese Parameter gewinnt man durch das erfindungsgemäße Verfahren.

Die erfindungsgemäße Vorrichtung zur Durchführung dieses Verfahrens ist bei einer ersten Ausführungsform dadurch gekennzeichnet, daß der Probentiegel zwei Probenkammern aufweist, die miteinander verbunden sind und in denen sich jeweils ein Temperatur-Meßfühler befindet, wobei die Wand der zweiten Probenkammer zumindest teilweise mit einem Sauerstoff abgebenden Mittel beschichtet ist. Man gibt somit die Gußeisenprobe in die unbeschichtete Kammer des Probentiegels, wobei sich die Probe in beiden Kammern des Tiegels über die Verbindung zwischen den beiden Kammern verteilt. Diese Vorrichtung gestattet somit auf konstruktiv einfache Weise die Durchführung des erfindungsgemäßen Verfahrens.

0157308

Eine zweite Ausführungsform einer solchen Vorrichtung ist in den Ansprüchen 3 und 4 gekennzeichnet.

Die Erfindung wird im folgenden anhand von Ausführungsbeispielen näher erläutert, aus denen sich weitere wichtige Merkmale ergeben. Es zeigt:

Fig. 1 - schematisch eine Draufsicht auf eine erste Ausführungsform einer erfindungsgemäßen Vorrichtung;

Fig. 2 - einen Schnitt längs der Linie A-A von Fig. 1;

Fig. 3 - eine Draufsicht entsprechend Fig. 1 bei einer zweiten Ausführungsform der erfindungsgemäßen Vorrichtung;

Fig. 4 - einen Schnitt längs B-B von Fig. 3;

Fig. 5 - ein Diagramm, wobei über der Zeit die Abkühlungskurve und die DDTA-Kurve einer Gußeisenprobe untereutektischer Zusammensetzung in einem herkömmlichen, unbeschichteten Tiegel dargestellt sind;

Fig. 6 - ein Diagramm entsprechend Fig. 5, wobei dieselbe Probe aber in einen Tiegel eingegeben wurde, dessen Innenwand mit einem Sauerstoff abgebenden Mittel beschichtet ist;

Fig. 7 - ein Schliffbild der Probe nach Fig. 5;

0157308

Fig. 8 - ein Schliffbild der Probe nach Fig. 6;

Fig. 9 - ein Diagramm, wobei über der zweidimensionalen x-Achse Analysendauer/Temperatur der gemessene Sauerstoffumsatz der Probe nach Fig. 5, 7 aufgetragen ist;

Fig.10 - ein Diagramm entsprechend Fig. 9 der Probe nach Fig. 6, 8.

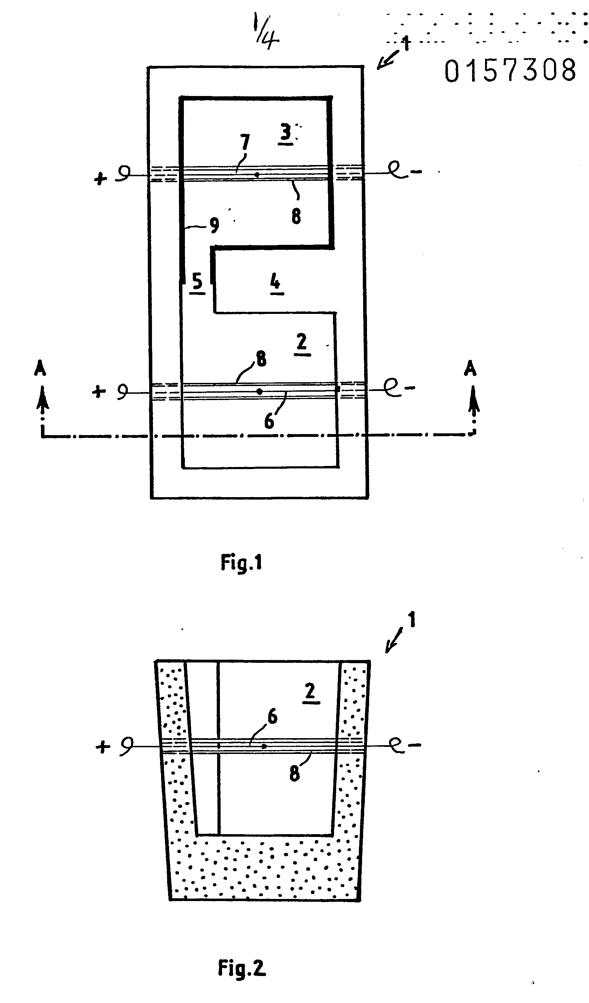

Fig. 1, 2 zeigen einen Probentiegel 1 mit zwei Probenkammern 2, 3. Beide Kammern sind durch eine Wand 4 voneinander getrennt, in der ein Durchlaß 5 zwischen den beiden Kammern ausgebildet ist. In der Probenkammer 2 befindet sich ein Temperatur-Meßfühler 6 und in der Probenkammer 3 ein weiterer Temperatur-Meßfühler 7. Beide Meßfühler sind durch die Wände der Probenkammern geführt und von einem Schutzröhrchen 8 umgeben.

Die zweite Probenkammer 3 ist erfindungsgemäß mit einer Beschichtung 9 aus einem geeigneten Sauerstoff abgebenden Material verbunden. Hierbei kann es sich um eine handelsübliche Schlichte mit Zusatz von Kupfer (II)oxid, Bleidioxid, Braunstein, Kaliumpermanganat, Soda oder Borax handeln.

Der Probentiegel 1 ist ein Einwegtiegel aus kunstharzgebundenem Sand.

Man gibt in die beiden Probenkammern 2, 3 eine Probe des zu untersuchenden Gußeisens ein und mißt in beiden Kammern die Abkühlungskurven über der Zeit mit Hilfe der beiden Meßfühlern. Daraus lassen sich die gesuchten Werte finden. Hierzu vergleiche weiter unten.

0157308

Gegebenenfalls kann man auch ausschließlich die Abkühlungskurve und/oder die DDTA-Kurve in der zweiten
Probenkammer 3 mit der Beschichtung 9 aus dem Sauerstoffabgebenenden Material messen oder auch die Reaktion
der Schmelze auf Sauerstoff und Impfmittel durch Zugabe
einer geeigneten Menge feingekörnten Impf- bzw. Desoxidationsmittels prüfen.

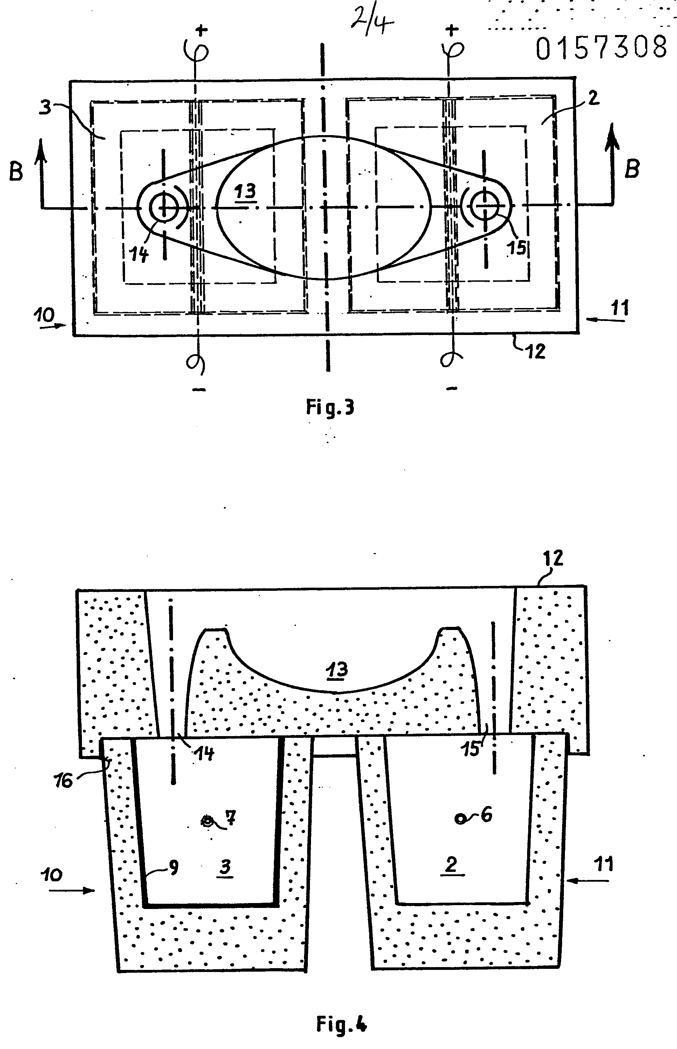

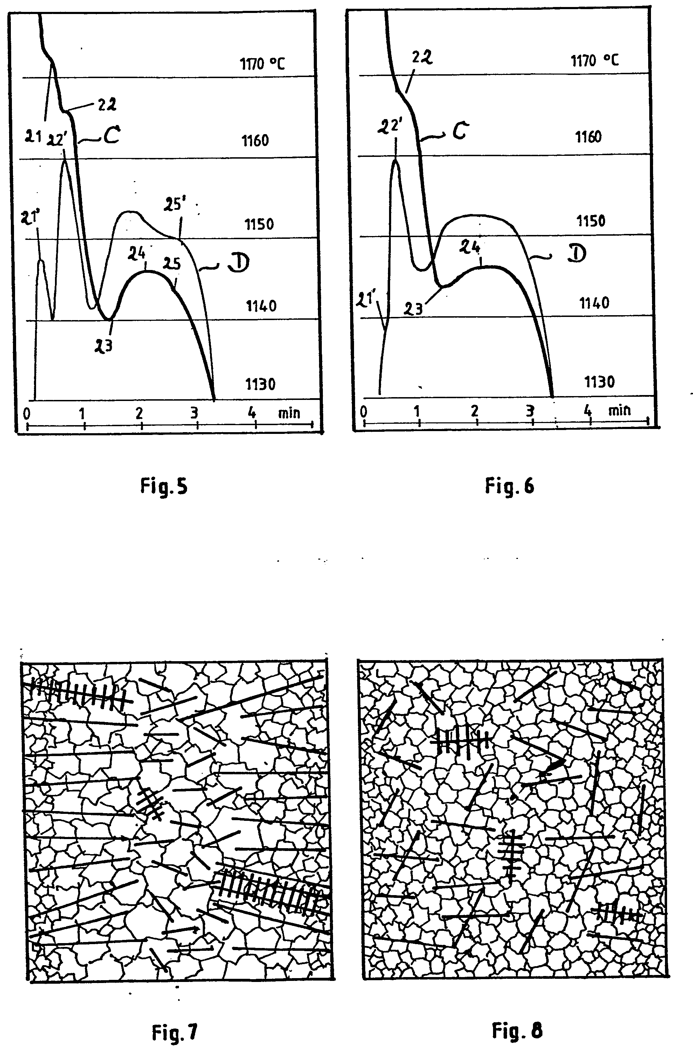

Die Fig. 3, 4 zeigen eine alternative Anordnung zur
durchführung des Meßverfahrens. Hier sind ein handelsüblicher, jedoch mit einem Sauerstoff abgebenden Mittel
9 beschichteter erster Probentiegel 10 und ein weiterer,
unbeschichteter Tiegel 11 nebeneinander angeordnet und mit
einer Abdeckplatte 12 aus chemisch gebundenem Sand
miteinander verbunden.

Die Abdeckplatte 12 enthält einen Eingußtümpel 13 mit zwei
Austrittsöffnungen 14 und 15. Das Eisen wird aus einem
Gießlöffel in den Tümpel 13 gegossen und betritt durch die
Öffnungen 14 und 15 gleichzeitig die mit der Sauerstoffabgebenden Beschichtung 9 versehene Kammer 3 sowie die
unbeschichtete Kammer 2 der Probentiegel 10 und 11. Dadurch ist gewährleistet, daß die Ausgangsparameter Temperatur
und Menge des Eisens in beiden Tiegeln gleich sind.

Die Erfahrung hat gezeigt, daß beim Füllen der Tiegel
nacheinander aus dem gleichen Gießlöffel lediglich
qualitativ auswertbare Ergebnisse zu erhalten wären,
nicht aber quantitativ auswertbare Meßergebnisse.

Die Abdeckplatte 12 kann an ihrer Unterseite mit einer
Aufnahme 16 versehen sein, in die die Tiegel 10, 11
hineinragen.

0157308

Im folgenden werden anhand von Fig. 5 - 10 die erhaltenen Meßergebnisse erläutert.

Insbesondere von Stahlguß, aber auch von Gußeisen ist bekannt, daß schlecht desoxidierte Schmelzen "exogen", d.h. unter Bildung von langen vom Rand zur Mitte eines Gußstücks ausgerichteten Primäraustenitdendriten, erstarren. Ursache dafür ist der Mangel an Kristallisationskeimen, d.h. an festen Desoxidationsprodukten, wie Tonerde und Kieselsäure, als Folge unzureichender Desoxidationsreserven. Unter solchen Bedingungen verbindet sich Sauerstoff anteilig stärker mit Eisen und Mangan zu FeO und MnO, die als Quelle für eine Reihe verdeckter Gußfehler, wie Randblasen, Schlackeneinschlüsse, Mikrolunker und Gefügeanomalien wie Weißeinstrahlung (Kantenhärte) und ungünstiger, d.h. im Wachstum gestörter Graphitformen, in Gußeisen erkannt wurden.

Die Abkühlungskurve einer Gußeisenschmelze enthält sämtliche Informationen über den Kristallisationsablauf. Sie äußern sich in der Größe und Abfolge nach Zeit und Temperatur der Knick- und Haltepunkte infolge Verzögerung der Wärmeabführung durch Abgabe der Kristallisationswärme bei der Ausscheidung einzelner Phasen. In der zeitlich richtigen Abfolge und der Menge ausgeschiedener Phasen proportionaler Größe läßt sich die jeweils abgegebene Kristallisationswärme durch die mit Hilfe eines optischen Meßverfahrens am Rande eines Gußstücks bzw. einer Probe durchgeführte DDTA erfassen. Aber auch ein Thermoelement mit mittig angeordneter Lötstelle ist in der Lage, Informationen über das Geschehen am Rand der Probe zu liefern. Zu diesem Zweck müssen die auf der Abkühlungskurve erkennbaren thermischen Effekte nur entsprechend umgedeutet werden:

Im Fall einer Gußeisenschmelze untereutektischer Zusammensetzung liefert die Abkühlungskurve bis zur beendeten Erstarrung zwei Hauptinformationen: die Liquidustemperatur TL als Beginn der Kristallisation des Primäraustenits in Probenmitte sowie die Solidus- bzw. eutektische Temperatur TE der Kristallisation des Austenit-Graphit-Eutektikums. Davor und danach müßte die Probe theoretisch nach dem Newton'schen Erkaltungsgesetz

$$t = \frac{m \cdot cp}{K} \, \ln \cdot \frac{T_1 - To}{T - To}$$

t = Zeit nach T

$T_1$ = Ausgangstemperatur

To = Endtemperatur (Raumtemperatur)

T = Temperatur nach Zeit t

m = Masse

cp = spez. Wärme

K = Proportionalitätskonstante

abkühlen. Weil sich in einer endlich großen Probe aber ein Temperaturgradient ausbildet und die Kristallisation am Rand beginnt, weicht der in Probenmitte gemessene Abkühlungsverlauf von diesem Gesetz ab.

Besonders ausgeprägt sind diese Abweichungen zu Beginn der Abkühlung, d.h. vor $T_L$. Die Größe der Abweichung Geschwindigkeit ihrer Änderung kann als Maß für den Erstarrungsablauf am Rand der Probe benutzt werden. Im Extremfall ist eine solche Abweichung als deutlicher Knickpunkt auf der Abkühlungskurve vor TL zu erkennen als Reaktion des mittigen Thermoelements auf die am Rand vehement beginnende Kristallisation und die plötzlich veränderten Wärmeabführungsbedingungen (Wärmestau). Die Temperaturdifferenz zwischen diesem Knickpunkt und TL in der Mitte ist praktisch gleich dem Temperaturgradienten zwischen Mitte und Rand der Probe zu diesem Zeitpunkt. Im Normalfall ist diese Abweichung auf der einfachen Abkühlungs-, d.h. Zeittemperatur-Kurve nicht ohne weiteres zu erkennen. Sie wird erst sichtbar, wenn gleichzeitig mit der Aufnahme der Abkühlungskurve eine K'-Analyse durchgeführt wird:

$$K' = \frac{m \cdot c_p}{K} \quad \text{bzw.}$$

$$K' = \frac{1}{t} \ln \frac{T_1 - T_o}{T - T_o} \, .$$

Wenn nach Maßgabe der K'-Änderung und Zunahme der fühlbaren Wärme des Probekörpers unter Berücksichtigung des Temperaturgradienten zwischen Mitte und Rand der Probe die Zeit-Temperatur-Kurve weiter analysiert wird, gelangt man zur quantitativen Erfassung der nach Zeit und Temperatur ausgeschiedenen Phasen (DDTA-Kurve).

In Kontakt mit Sauerstoff im mit einem Sauerstoff abgebenden Mittel beschichteten Tiegel werden in der Schmelze nur dann neue Keime gebildet, wenn sie über eine hinreichende Desoxidationsreserve verfügt. Der aufgenommene Sauerstoff wird dabei in der Regel als Tonerde und Kieselsäure gebunden. Ist eine Desoxidationsreserve nicht vorhanden, wird die Bildung von FeO und MnO gegenüber der Probe im unbeschichteten Tiegel verstärkt.

Fig. 5 zeigt die Abkühlungskurve C und die DDTA-Kurve D einer Gußeisenprobe untereutektischer Zusammensetzung im unbeschichteten Tiegel. Sie zeigt oberhalb der Liquidustemperatur 22 einen weiteren Knickpunkt 21, der einer heftigen Primärkristallisation am Rand zuzuordnen ist. (Kurve C). Entsprechend deutlich ist der Peak 21' auf der DDTA-Kurve D ausgebildet, deren Fläche der ausgeschiedenen Primäraustenitmenge proportional ist.

Die vergleichsweise hohe Differenz zwischen unterer 23 und oberer 24 eutektischer Temperatur TE (Unterkühlung) ist ein Hinweis auf eine Störung der Graphitkristallisation (Kurve C). Diese Störung offenbart sich auch im Knickpunkt 25 im Verlauf der eutektischen Kristallisation, durch den die Erstarrung einer gesteigerten Restschmelze signalisiert wird. Dem Knickpunkt 25 entspricht der Knickpunkt 25' der Kurve D.

Die Schmelze erstarrte also "exogen", d.h. unter Bildung langer gerichteter Primäraustenitdendriten bei vergleichsweise grobem eutektischen Korn. Dies zeigt Fig. 7.

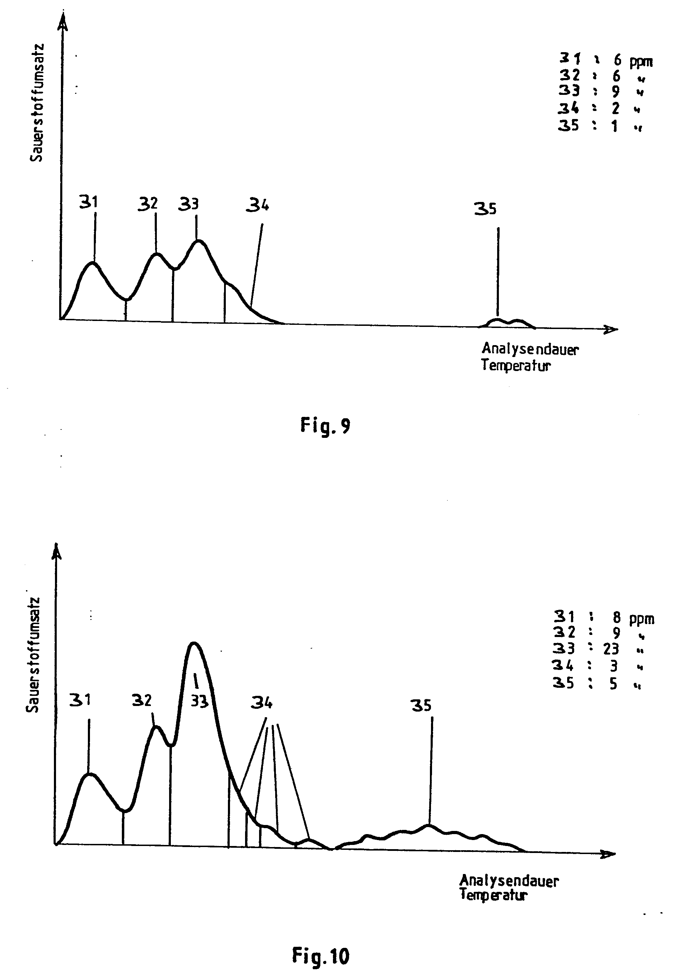

Der Mangel an Kristallisationskeimen ist ersichtlich aus Fig. 9. Ohne Berücksichtigung des molekular an der Oberfläche angelagerten Sauerstoffs 31 zeigt die Oxidspektrumanalyse, daß von insgesamt 18 ppm $O_2$ nur 1 ppm als Tonerde 35 (Soll : mindestens 5 ppm) und 9 ppm als Kieselsäure 33 gebunden sind. Das Verhältnis von $SiO_2$ (33) zu FeO+MnO (32) beträgt dabei 1,5 (=9ppm : 6 ppm). Ungestörte $SiO_2$ -, d.h. Kristallisationskeimbildung ist aber erfahrungsgemäß nur gegeben, wenn dieses Verhältnis größer oder gleich 2.

Fig. 6 zeigt die Abkühlungskurve C und DDTA-Kurve D der gleichen Schmelze im mit einem Sauerstoff abgebenden Mittel beschichteten Tiegel. Die Abweichung von K' ist auf der

0157308

Abkühlungskurve C oberhalb TL (22) nicht sichtbar, auf der DDTA-Kurve D ist sie jedoch durch den Knick 21' gekennzeichnet. Die Unterkühlung, d.h. die Temperaturdifferenz zwischen oberer 24 und unterer 23 eutektischen Temperatur, ist kleiner geworden, der Knickpunkt 25 in Fig. 5 fehlt. Die Schmelze erstarrte "endogen" unter Bildung kleiner, regellos verteilter Primäraustenit-Dentriten bei vergleichsweise feinem eutektischen Korn (Fig. 8).

Die Oxidspektrumanlayse Fig. 10 der Probe weist bei einem Gesamtsauerstoffgehalt von 40 ppm (ohne Berücksichtigung des sogenannten Oberflächensauerstoffs 31) einen Sauerstoffzuwachs von 22 ppm überwiegend zugunsten der Tonerde 35 (5 ppm) und Kieselsäure 33 (23 ppm). Das Verhältnis $SiO_2$ (33.) zu FeO+MnO (32) ist mit 2,56 (= 23 ppm:9 ppm) als Hinweis auf ungestörte Kristallisationskeimbildung zu deuten.

Im vorliegenden Fall hatte die Schmelze genügend Desoxidationsreserve, um auch ohne eine Impfung in der Form ungestört zu erstarren. Das Impfen, d.h. die Zugabe eines Desoxidationsmittels, führte möglicherweise zu einer Überdesoxidation mit den bekannten nachteiligen Wirkungen. Die Spitzen 21' und 25' der DDTA-Kurve D in Fig. 5 können somit als Parameter für die Wahl der Schmelzbehandlungsmethode genommen werden. Eine relative Verstärkung dieser Spitzen in Kontakt mit Sauerstoff würde umgekehrt einen Impfmittelbedarf signalisieren. Die erforderliche Menge kann aus der Größe der auftretenden Differenz abgeleitet werden.

0157308

Patentansprüche

1. Verfahren zur thermischen Analyse von Gußeisen,
   wobei man eine Probe des zu untersuchenden flüssigen
   Gußeisens in einen Probentiegel (1) mit einem Temperatur-Meßfühler (6) gibt und die Abkühlung der Probe über
   der Zeit mißt,
   d a d u r c h   g e k e n n z e i c h n e t,
   daß man dieselbe Probe gleichzeitig in einen zweiten
   Probentiegel (3) mit einem zweiten Temperatur-Meßfühler
   (7) gibt, dessen Innenwand zumindest teilweise mit
   einem Sauerstoff abgebenden Mittel beschichtet ist.

2. Vorrichtung zur Durchführung des Verfahrens nach
   Anspruch 1 mit einem Probentiegel mit einem Temperatur-
   Meßfühler,
   d a d u r c h   g e k e n n z e i c h n e t,
   daß der Probentiegel (1) zwei Probenkammern (2, 3)
   aufweist, die miteinander verbunden sind und in denen
   sich jeweils ein Temperatur-Meßfühler (6, 7) befindet,
   wobei die Wand der zweiten Probenkammer (3) zumindest
   teilweise mit einem Sauerstoff abgebenden Mittel (9)
   beschichtet ist.

3. Vorrichtung zur Durchführung des Verfahrens nach
   Anspruch 1 mit einem Probentiegel mit einem Temperatur-
   Meßfühler,
   d a d u r c h   g e k e n n z e i c h n e t,
   daß neben dem Probentiegel (11) ein weiterer Probentiegel (10) mit einem weiteren Temperatur-Meßfühler
   angeordnet ist, dessen Innenwand zumindest teilweise
   mit einem Sauerstoff abgebenden Mittel (9) beschichtet
   ist, wobei für beide Probentiegel (10, 11) eine gemeinsame Abdeckplatte (12) mit einem Eingußtümpel (13)

0157308

vorgesehen ist, der mit Öffnungen (14, 15) in Verbindung steht, die zu den beiden Probentiegeln (10, 11) führen.

4. Vorrichtung nach Anspruch 3,
   d a d u r c h   g e k e n n z e i c h n e t ,
   daß die Abdeckplatte (12) an der Unterseite eine Aufnahme (16) für die beiden Probentiegel (10, 11) hat.

Der Patentanwalt:


Dr. D. Gudel

Fig.1

Fig.2

0157308

Fig.3

Fig.4

3/4 0157308

Fig. 5

Fig. 6

Fig. 7

Fig. 8

31 ν 6 ppm
32 ι 6 „
33 ι 9 „
34 ι 2 „
35 ι 1 „

Fig. 9

31 ι 8 ppm
32 ι 9 „
33 ι 23 „
34 ι 3 „
35 ι 5 „

Fig. 10